# EUROPÄISCHE PATENTSCHRIFT

(11) **EP 3 119 292 B1**
(45) Veröffentlichungstag und Bekanntmachung des Hinweises auf die Patenterteilung: **16.08.2023**
(21) Anmeldenummer: 15709151.3
(22) Anmeldetag: 10.03.2015
(51) Int. Cl.: A61B 17/221, A61F 2/91, A61B 17/00

(54) **FANGDRAHTINSTRUMENT MIT FANGDRAHTSTRUKTUR AUS ROHRSTÜCK**
SNARE INSTRUMENT WITH SNARE STRUCTURE COMPOSED OF A TUBULAR PIECE
INSTRUMENT À FIL DE CAPTURE AVEC STRUCTURE DE FIL DE CAPTURE CONSTITUÉE D'UNE PIÈCE TUBULAIRE

(30) Priorität: 21.03.2014 DE 102014205366
(43) Veröffentlichungstag der Anmeldung: 25.01.2017
(73) Patentinhaber: Coloplast A/S, 3050 Humlebaek (DK)
(72) Erfinder: UIHLEIN, Bernhard, 72581 Dettingen (DE)
(86) Internationale Anmeldenummer: PCT/EP2015/054894
(87) Internationale Veröffentlichungsnummer: WO 2015/139998

(56) Entgegenhaltungen:
- WO-A1-03/075793
- WO-A1-2013/071115
- WO-A2-2007/103236
- WO-A2-2010/034021
- US-A1- 2005 165 441
- US-A1- 2006 009 785
- US-A1- 2011 319 917
- US-A1- 2012 116 440
- US-A1- 2012 116 496
- US-A1- 2013 289 578

## Beschreibung

Die Erfindung bezieht sich auf ein Fangdrahtinstrument nach dem Oberbegriff des Anspruchs 1.

Derartige Fangdrahtinstrumente werden insbesondere als medizinische Fangdrahtinstrumente verwendet, um Fremdkörper, Blutklumpen, Steine, oder andere Ablagerungen aus menschlichen oder tierischen Geweben entfernen bzw. abfangen zu können, vorzugsweise unter Benutzung eines entsprechenden Katheterinstruments.

Bei einem bekannten Typ solcher Instrumente hat der aufgefaltete Einfangabschnitt eine Korbform, d.h. er bildet einen Fangkorb, der zusammengefaltet in der Umhüllung aufgenommen und aus dieser nach vorn herausbewegt werden kann, wodurch er sich zu seiner Korbform auffaltet. Steine und dergleichen können dann quer zur Längsachse des Drahtkorbs im Bereich von dessen maximalem Durchmesser und damit maximaler Öffnungsweite in den Drahtkorb eingefangen werden. Denn in diesem Bereich haben benachbarte Drahtabschnitte der korbbildenden Fangdrahtstruktur ihren maximalen Abstand, wodurch dort korbumfangsseitig entsprechende Einfangöffnungen gebildet sind. Durch wenigstens teilweises Zurückziehen des Drahtkorbs in die Umhüllung wird dieser eingefaltet, bis er das eingefangene Objekt eng umschließt und dieses dadurch festhält. Anschließend kann der Drahtkorb samt Umhüllung und eingefangenem Objekt aus dem betreffenden Gewebekanal herausbewegt werden. Steinfangkorb- bzw. Ballonkatheterinstrumente dieser Art sind z.B. in der Patentschrift EP 1 809 187 B1 und der Offenlegungsschrift DE 197 22 429 A1 offenbart.

Ein weiterer bekannter Typ solcher Fangdrahtinstrumente sind sogenannte Fangschlingeninstrumente, bei denen die Fangdrahtstruktur aus einer oder mehreren zusammenwirkenden Drahtschlingen gebildet ist, die frontseitig lose oder gefasst sein können, siehe z.B. die Offenlegungsschrift WO 02/078632 A2.

In den Offenlegungsschriften US 2005/0165442 A1 und US 2005/0165441 A1 ist jeweils eine Filterdrahtstruktur offenbart, die dafür ausgelegt ist, bleibend in einem Gewebekanal, insbesondere einem Blutgefäß, platziert zu werden, um Partikel bzw. Blutklumpen abzufangen und dadurch Embolien vorzubeugen. Dazu wird ein Kathetherrohr, in welchem die Filterdrahtstruktur eingefaltet aufgenommen ist, in den Gewebekanal eingeführt, wonach die Filterdrahtstruktur mittels eines Schiebedrahtes aus dem Kathetherrohr herausgedrückt wird, das dann ebenso wie der Schiebedraht wieder aus dem Gewebekanal herausbewegt wird. Die Filterdrahtstruktur ist durch Schlitzen und Aufbiegen einteilig aus einem Rohrstück gebildet und weist eine Kelchform auf, die am distalen Ende mit V-förmigen, nach vorn und leicht radial nach außen weisenden Spitzen abschließt. Diese V-förmig zugespitzten distalen Kelchenden resultieren aus der zugehörigen speziellen Schlitzung des Rohrstücks in seinem äußersten distalen Endabschnitt. Vom distalen Rohrstückende her eingebrachte, offene axiale Schlitze wechseln sich mit zwischenliegenden, mit Abstand vor dem distalen Rohrstückende endenden, geschlossenen axialen Schlitzen ab, wobei in einem axial überlappenden Bereich die offenen und die geschlossenen axialen Schlitze etwa gleich breit und deutlich schmaler sind als die dadurch zwischen den Schlitzen aus dem Rohrstück gebildeten Drahtstücke. Am distalen Ende erstrecken sich die offenen Schlitze mit einer konischen Aufweitung nach vorn, so dass der Radius eines Krümmungsbereichs, über den am distalen Ende je zwei der Drahtstücke zusammenhängen, höchstens etwa so groß ist wie die dortige Breite der Drahtstücke. Dadurch bleibt die sich nach vorn zuspitzende V-Form dieser Drahtstückverbindungen auch nach dem Aufbiegen der Filterdrahtstruktur erhalten. Bei Bedarf ist am proximalen Ende der Filterdrahtstruktur ein Fanghaken ausgebildet, um sie mittels eines Rückholfangdrahtes wieder aus dem Gewebekanal herausholen zu können.

Dokumente US2006/0009785 A1, WO 2010/034021 A2, WO 03/075793 A1 und US 2012/0116440 A1 offenbaren weitere verschiedene Fangdrahtinstrumente mit einer Fangdrahtstruktur.

Der Erfindung liegt als technisches Problem die Bereitstellung eines Fangdrahtinstruments der eingangs genannten Art zugrunde, das eine gute Fangfunktionalität und hohe Funktionszuverlässigkeit bietet und sich mit vergleichsweise geringem Aufwand fertigen lässt.

Die Erfindung löst dieses Problem durch die Bereitstellung eines Fangdrahtinstrumentes mit den Merkmalen des Anspruchs 1. Weitere bevorzugte Ausführungsformen werden in den Unteransprüchen dargelegt. Bei diesem erfindungsgemäßen Fangdrahtinstrument weist der Einfangabschnitt im aufgefalteten Zustand eine Kelchform mit distaler Einfangöffnung auf. Unter Einfangöffnung ist dabei vorliegend der Bereich maximaler Öffnungsweite der Fangdrahtstruktur zu verstehen, der dazu bestimmt und eingerichtet ist, durch ihn hindurch einzufangende Objekte in das Innere der Fangdrahtstruktur hineingelangen zu lassen. Zu diesem Zweck sind in einen distalen Endbereich des zur Herstellung der Fangdrahtstruktur verwendeten Rohrstücks vom Rohrstückende her offene axiale Schlitze geeigneter Länge sowie zwischenliegende, mit Abstand vor dem distalen Rohrstückende endende, geschlossene axiale Schlitze eingebracht, wobei die geschlossenen axialen Schlitze länger als die offenen axialen Schlitze sind und sich daher in proximaler Richtung über die offenen axialen Schlitze hinaus erstrecken. Die verbleibenden Rohrstückbereiche zwischen den offenen und geschlossenen axialen Schlitzen bilden Drahtstücke, die durch das Aufbiegen des Rohrstücks Umfangsabschnitte der Einfangöffnung bilden. Der Einfangabschnitt kann von seinem aufgefalteten, kelchförmigen Zustand bei Bedarf wieder vollständig in eine dem Rohrstück entsprechende Form eingefaltet bzw. zusammengefaltet werden, wenn die Fangdrahtstruktur vollständig in die Umhüllung zurückbewegt wird.

Mit dem erfindungsgemäßen Fangdrahtinstrument können Steine und andere Partikel in Körpergeweben in einer vorteilhaften Weise von vorn eingefangen werden, d.h. sie gelangen von vorn durch die distale Einfangöffnung in axialer Richtung in den kelchförmig aufgefalteten Einfangabschnitt. Dazu kann die Fangdrahtstruktur entsprechend axial vorbewegt werden. Auf diese Weise lassen sich auch Partikel, die sich in distaler Richtung nahe einer Gewebewand befinden, problemlos einfangen. Das erfindungsgemäße Instrument kann auch dazu verwendet werden, nach Art eines Filters oder Stents Fremdkörper aus Blutbahnen und anderen Körpergefäßen zu entfernen bzw. abzufangen.

Das erfindungsgemäße Fangdrahtinstrument lässt sich mit relativ geringem Aufwand herstellen, indem seine Fangdrahtstruktur einteilig aus einem Rohrstück gebildet wird, das zu diesem Zweck in an sich bekannter Weise geschlitzt und aufgebogen wird. Schweißstellen zum Verbinden von Drahtstücken können entfallen. Indem die am distalen Rohrstückende offen ausmündenden Schlitze eine geeignete Länge aufweisen, können die Drahtstücke, die aus dem Rohrstück durch die offenen und geschlossenen axialen Schlitze in deren axialem Überlappungsbereich gebildet sind, den Umfang der Einfangöffnung bilden, d.h. den distalen Rand der Kelchform des aufgefalteten Einfangabschnitts. Durch die Länge der offenen axialen Schlitze lässt sich somit die Umfangslänge und damit auch der Einfangquerschnitt bzw. die Öffnungsweite des Einfangkelches vorgeben. Mit größerer Länge der offenen axialen Schlitze kann die Umfangslänge und damit der Einfangquerschnitt des Einfangkelches erhöht werden. Da sich die geschlossenen axialen Schlitze proximal über die offenen axialen Schlitze hinaus erstrecken, teilen sie das Rohrstück in dem proximalen Bereich hinter den offenen axialen Schlitzen in hintere Drahtstücke, die im aufgefalteten Zustand einen Kelchkörper für den Einfangkelch bilden, wobei dieser Kelchkörper an seinem distalen Ende wie erwähnt mit den durch die vorderen Drahtstücke gebildeten Kelchrand abschließt. Dabei verzweigt sich jedes hintere Drahtstück in zwei vordere Drahtstücke.

In einer Weiterbildung der Erfindung hängen je zwei benachbarte vordere Drahtstücke, die aus dem Rohrstück durch die offenen und geschlossenen axialen Schlitze gebildet sind, über einen Krümmungsbereich zusammen, dessen Radius R_{D} mindestens so groß ist wie das Zweieinhalbfache einer Breite b_{D} der Drahtstücke im Krümmungsbereich, d.h. R_{D} ≥ 2,5×b_{D}. Es zeigt sich, dass sich die Drahtstücke durch diese Dimensionierungsmaßnahme sehr vorteilhaft unter Bildung des Kelchrandes aufbiegen lassen, ohne dass Bruchgefahr besteht, wobei spitz zulaufende Kelchrandbereiche vermieden werden, die im Gebrauch zu Gewebeverletzungsrisiken führen könnten.

In einer Weiterbildung der Erfindung beträgt eine Umfangslänge U der Einfangöffnung 70% oder mehr des Produkts aus der Anzahl 2n der durch die offenen und geschlossenen axialen Schlitze aus dem Rohrstück gebildeten Drahtstücke multipliziert mit der Länge L der offenen axialen Schlitze, d.h. U ≥ 0,7×2nL. Diese Dimensionierungsmaßnahme ermöglicht die Bereitstellung eines vergleichsweise großen Einfangquerschnitts mit einem Kelchrand, der in axialer Richtung allenfalls stumpfwinklige und keine spitzwinkligen V-förmigen Abschnitte aufweist.

Je nach dem verwendeten Aufbiegevorgang lassen sich in Weiterbildung der Offenbarung unterschiedliche Kelchformen für den Einfangabschnitt im aufgefalteten Zustand mit Bereichen erzielen, in denen der Kelchdurchmesser in distaler Richtung linear, progressiv oder degressiv zunimmt.

Entsprechend der Erfindung ist ein maximaler Querschnitt der Kelchform des Einfangabschnitts im aufgefalteten Zustand größer als ein Querschnitt der Einfangöffnung. Dies kann in bestimmten Anwendungsfällen das Festhalten eines eingefangenen Partikels im eingefalteten Einfangkelch erleichtern.

In einer Weiterbildung der Erfindung umfasst der Einfangabschnitt weitere, zweite geschlossene axiale Schlitze im Rohrstück, die sich nach vorn mit ihrem distalen Ende auf Höhe zwischen einem distalen und einem proximalen Ende der ersten geschlossenen axialen Schlitze und nach hinten mit ihrem proximalen Ende hinter dem proximalen Ende der ersten geschlossenen axialen Schlitze erstrecken. Dies ermöglicht eine drahtverzweigende Konfiguration des in diesem Fall von den ersten und zweiten geschlossenen axialen Schlitzen gebildeten Kelchkörpers.

In einer Weiterbildung der Erfindung ist die Fangdrahtstruktur und/oder die Umhüllung und/oder der in der Umhüllung axial beweglich geführter Zugdraht für die Fangdrahtstruktur ganzflächig oder teilflächig oder punktuell mit einem Magnetresonanz(MR)-Markermaterial versehen. Dies ermöglicht eine vorteilhafte Verwendung des Fangdrahtinstrumentes in entsprechenden MR-Anwendungen, indem seine Fangdrahtstruktur MR-sichtbar ist.

Vorteilhafte Ausführungsformen der Erfindung sind in den Zeichnungen dargestellt und werden nachfolgend beschrieben. Hierbei zeigen:
- Fig. 1: eine schematische Längsschnittansicht eines Fangdrahtinstruments mit kelchförmigem Einfangabschnitt,
- Fig. 2: eine perspektivische Ansicht eines Einfangabschnitts einer Fangdrahtstruktur des Instruments von Fig. 1,
- Fig. 3: eine schematische Seitenansicht des Einfangabschnitts von Fig. 2 zur Erläuterung eines Partikeleinfangvorgangs,
- Fig. 4: eine Abwicklungsdarstellung eines geschlitzten Rohrstücks für die Fangdrahtstruktur des Instruments von Fig. 1,
- Fig. 5: eine Detailansicht eines distalen Drahtstück-Verbindungsbereichs des geschlitzten Rohrstücks von Fig. 4,
- Fig. 6: die Ansicht von Fig. 5 nach Aufbiegen des geschlitzten Rohrstücks zur Bildung des kelchförmigen Einfangabschnitts,
- Fig. 7: eine Draufsicht von vorn auf den kelchförmigen Einfangabschnitt,
- Fig. 8: eine schematische Seitenansicht einer Fangdrahtstruktur mit in distaler Richtung degressiv zunehmendem Durchmesser des Einfangkelchs,
- Fig. 9: eine Ansicht entsprechend Fig. 8 für eine Fangdrahtstruktur mit in distaler Richtung linear zunehmendem Einfangkelchdurchmesser,
- Fig. 10: eine Ansicht entsprechend Fig. 8 für eine Fangdrahtstruktur mit distaler Richtung progressiv zunehmendem Einfangkelchdurchmesser,
- Fig. 11: eine Draufsicht entsprechend Fig. 7 auf einen anderen Einfangkelch mit drei statt vier hinteren Kelchkörper-Drahtstücken und mit ganzflächiger MR-Markerbeschichtung,
- Fig. 12: eine Ansicht entsprechend Fig. 11 für eine Variante mit teilflächig und punktuell aufgebrachtem MR-Markermaterial,
- Fig. 13: eine Detailansicht von Fig. 12 eines Bereichs mit teilflächigem MR-Markermaterial,
- Fig. 14: eine Detailansicht von Fig. 12 mit punktuell aufgebrachtem MR-Markermaterial,
- Fig. 15: eine Detailschnittansicht des Instruments von Fig. 1 mit MR-Markermaterial in einem Schaftbereich und
- Fig. 16: eine Seitenansicht einer Umhüllung mit MR-Markermaterial, wie sie für das Instrument von Fig. 1 verwendbar ist.

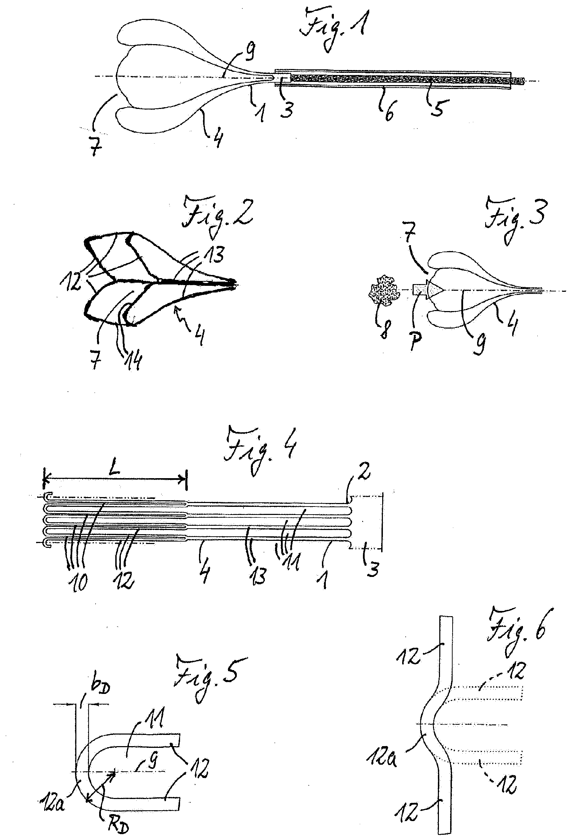

Das in den Fig. 1 bis 7 gezeigte Fangdrahtinstrument weist eine Fangdrahtstruktur 1 auf, die durch Schlitzen und Aufbiegen einteilig aus einem in seiner Abwicklungsdarstellung in Fig. 4 gezeigten Rohrstück 2 gebildet ist. Die Fangdrahtstruktur 1 umfasst einen proximalen, ungeschlitzten Basisabschnitt 3 und einen sich davon axial nach vorn erstreckenden distalen Einfangabschnitt 4.

Die Fangdrahtstruktur 1 ist an ihrem proximalen Endbereich mit einem distalen Endbereich eines als Zugdraht bezeichneten Betätigungselements 5 gekoppelt, das in einer Umhüllung 6 axial beweglich geführt ist. Die Kopplung ist vorzugsweise dergestalt realisiert, dass der proximale Endbereich der Fangdrahtstruktur 1 bleibend und axial bewegungsstarr mit dem distalen Endbereich des Betätigungselements 5 verbunden ist, z.B. durch eine Klebe- oder Schweißverbindung. Dadurch kann die Fangdrahtstruktur 1 mittels entsprechender axialer Betätigungsbewegung des Betätigungselements 5 sowohl axial nach vorn als auch axial nach hinten bewegt werden. Zugdraht 5 und Umhüllung 6 sind vorzugsweise aus einem elastisch biegefähigen Material gefertigt. In Fig. 1 ist die Fangdrahtstruktur 1 in einer Einfangposition gezeigt, in welcher sie vollständig aus der Umhüllung 6 nach vorn herausbewegt ist und der Einfangabschnitt 4 einen vollständig aufgefalteten Zustand einnimmt. Durch Zurückziehen des Zugdrahtes 5 und/oder Vorschieben der Umhüllung 6 kann die Fangdrahtstruktur 1 vollständig in die Umhüllung 6 eingezogen werden, wobei sich ihr Einfangabschnitt 4 zusammenfaltet, d.h. bis auf den Innendurchmesser der Umhüllung 6 einfaltet. Der Einfangabschnitt 4 nimmt in dieser vollständigen Einfaltposition ungefähr die in Fig. 4 gezeigte, anfängliche Gestalt an, wie sie nach dem Schlitzen und vor dem Aufbiegen des Rohrstücks 3 vorliegt. Die Techniken zum Schlitzen und Aufbiegen des Rohrstücks 3 zwecks Erzielung der Fangdrahtstruktur 1 sind dem Fachmann geläufig. Für die Fangdrahtstruktur 1 werden dazu geeignete Materialien verwendet, die sich elastisch aufbiegen und durch einen Behandlungsprozess, wie Backen oder Härten, elastisch im aufgebogenen Zustand fixieren lassen. Beispielsweise sind hierfür superelastische Materialien, wie NiTi-Legierungen, gebräuchlich.

Wie insbesondere aus den Fig. 1 bis 3 ersichtlich, besitzt der Einfangabschnitt 4 im aufgefalteten Zustand eine Kelchform mit distaler Einfangöffnung 7, die gleichzeitig die maximale Öffnungsweite der Fangdrahtstruktur 1 darstellt. Entsprechend dient dieses Fangdrahtinstrument in medizinischen Anwendungen, wie als Steinfanginstrument, dazu, Steine oder andere Partikel im Körpergewebe eines Patienten von vorn in den Einfangabschnitt 4 einzufangen. Dies unterscheidet das Instrument von herkömmlichen Fangkorbinstrumenten, bei denen die Partikel quer zur Instrumentenlängsachse, d.h. in radialer Richtung, in einen vorne und hinten weitgehend geschlossenen Drahtkorb eingefangen werden.

Fig. 3 veranschaulicht ein solches Einfangen eines Partikels 8, z.B. eines Nierensteins, von vorn durch die Einfangöffnung 7 hindurch in proximaler Richtung P parallel zu einer Längsachse 9 des Instruments und damit auch des kelchförmigen Einfangabschnitts 4. Dies kann den Einfangvorgang im Vergleich zu Instrumenten mit vorn und hinten geschlossenem Fangkorb erleichtern, da das Instrument für den Einfangvorgang primär nur axial und nicht in Querrichtung bewegt werden braucht. Zudem kann dies das Einfangen von Partikeln in Fällen erleichtern, in denen sich die Partikel in axialer Richtung nahe vor einer dahinterliegenden Gefäßwand befinden.

Wie insbesondere aus den Fig. 2 und 4 ersichtlich, wird das Rohrstück 2 beim Schlitzen mit einer speziellen Schlitzstruktur versehen, die am distalen Rohrstückende offen ausmündende Schlitze 10 und zwischenliegende, mit Abstand vor dem distalen Rohrstückende endende, geschlossene axiale Schlitze 11 umfasst. Die geschlossenen axialen Schlitze 11 erstrecken sich in proximaler Richtung über die distal offenen Schlitze 10 hinaus nach hinten. Durch diese Schlitzstruktur sind aus dem betreffenden distalen Rohrstückabschnitt erste, vordere Drahtstücke 12 und zweite, hintere Drahtstücke 13 gebildet. Die vorderen Drahtstücke 12 liegen hierbei in einem axialen Bereich vor, in welchem sich die offenen Schlitze 10 und die geschlossenen Schlitze 11 überlappen, wobei sich die offenen und die geschlossenen Schlitze 10, 11 in Umfangsrichtung abwechseln. Die hinteren Drahtstücke 13 liegen in dem axialen Bereich vor, in welchem sich die geschlossenen Schlitze 11 proximal über die offenen Schlitze 10 hinaus erstrecken. Dabei verzweigt sich am Übergangsbereich jedes hintere Drahtstück 13 in zwei vordere Drahtstücke 12.

Der Abstand, mit dem die geschlossenen Schlitze 11 vor dem distalen Rohrstückende enden, definiert eine Drahtstückbreite b_{D} eines gekrümmten Drahtstückbereichs 12a, über den jedes vordere Drahtstück 12 mit einem benachbarten vorderen Drahtstück 12 distal zusammenhängt. Die Drahtstückbreite b_{D} dieses im gezeigten Beispiel halbkreisförmig gekrümmten Drahtverbindungsbereichs 12a kann z.B. in etwa der Breite der damit verbundenen vorderen Drahtstücke 12 entsprechen, wie dies aus Fig. 5 erkennbar ist. Weiter ist aus Fig. 5 ersichtlich, dass ein Krümmungsradius R_{D} dieses gekrümmten Drahtverbindungsbereichs 12a wesentlich größer ist als der Durchmesser bzw. die Breite b_{D} des Drahtverbindungsbereichs 12a. Durch den entsprechenden Schlitzvorgang wird dafür gesorgt, dass dieser Krümmungsradius R_{D} mindestens das Zweieinhalbfache dieser Drahtstückbreite b_{D} beträgt, d.h. R_{D}≥2,5×b_{D}. Dies hat zur Folge, dass sich beim Aufbiegen des geschlitzten Rohrstücks in die Kelchform des Einfangabschnitts 4 die vorderen Drahtstücke 12 in ihrem gekrümmten Verbindungsbereich 12a bis zu einem gewünschten Aufbiegungswinkel, der vergleichsweise groß sein kann, aufbiegen lassen, ohne dass die Gefahr von Brüchen im Drahtverbindungsbereich 12a entsteht, wobei sich bei Bedarf jegliche in distaler Richtung spitz zulaufende Endkonturen für die Fangdrahtstruktur 1 vermeiden lassen. Fig. 6 veranschaulicht beispielhaft das Aufbiegen zweier über den Verbindungsbereich 12a verbundener Drahtstücke 12 aus ihrer gestrichelt wiedergegebenen, parallelen Ausgangslage in eine auf 180° aufgebogene Stellung.

Die erwähnte Schlitzstruktur des Rohrstücks 2 führt dazu, dass sich der Einfangabschnitt 4 in gewünschter Weise zu der erwähnten Kelchform mit der distalen Einfangöffnung 7 aufbiegen lässt. Dabei bilden, wie aus Fig. 2 zu erkennen, die hinteren Drahtstücke 13 einen Kelchkörper, während die vorderen Drahtstücke 12 einen distalen Umfangsrand 14 des Einfangkelchs 4 bilden, d.h. den Rand der distalen Einfangöffnung 7. Somit bestimmt bzw. begrenzt die Länge der vorderen Drahtstücke 12 und die Anzahl derselben die maximale Länge des distalen Randes des Einfangkelchs 4. Mit anderen Worten werden die distal offenen Schlitze 10, wie in Fig. 4 gezeigt, in einer geeigneten Länge L in das Rohrstück 2 eingebracht, die so gewählt ist, dass sich der Einfangabschnitt 4 in die gewünschte Kelchform aufbiegen lässt, wobei sich die Länge des Kelchrandes und damit auch der Umfang des effektiven Eintrittsquerschnitts der Eintrittsöffnung 7 durch diese Länge L der offenen Schlitze 10 und damit der vorderen Drahtstücke 12 sowie durch das Maß an Aufbiegung der vorderen Drahtstücke 12 an ihrem Drahtverbindungsbereich 12a bestimmt.

Im gezeigten Beispiel von Fig. 4 beinhaltet die Schlitzstruktur je vier distal offene Schlitze 12 und vier geschlossene Schlitze 11 und folglich eine Anzahl n von vier hinteren Drahtstücken 11 und eine doppelt so große Anzahl 2n von acht vorderen Drahtstücken 12. Es versteht sich, dass in alternativen Ausführungsformen der Erfindung eine beliebige Anzahl n von geschlossenen bzw. distal offenen Schlitzen vorgesehen sein kann, d.h. die Anzahl n kann eine beliebige ganze Zahl größer als eins sein.

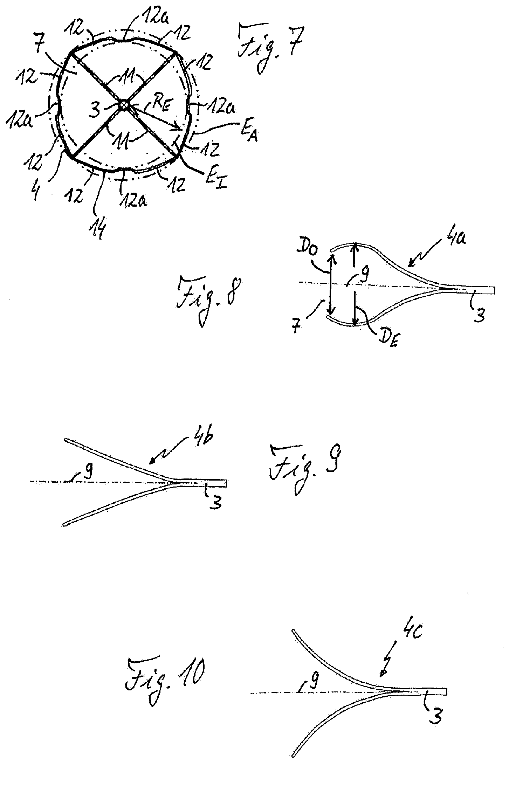

Die erwähnten Dimensionierungsverhältnisse lassen sich anhand der Draufsicht auf den Einfangkelch 4 bzw. dessen Einfangöffnung 7 näher erläutern, wie sie in Fig. 7 wiedergegeben ist. Wie aus Fig. 7 für den beispielhaften Fall einer Anzahl n=4 von offenen bzw. geschlossenen Rohrschlitzen und damit einer Anzahl n=4 von vier hinteren Drahtstücken 11 und einer doppelten Anzahl 2n=8 von vorderen Drahtstücken 12 zu erkennen, wird der Kelchrand 14 durch die acht vorderen Drahtstücke 12 zuzüglich deren vier Verbindungsbereiche 12a gebildet. Entsprechend beträgt die Länge dieses Kelchrandes etwa das Achtfache der Länge L eines vorderen Drahtstückes 12 zuzüglich eines demgegenüber deutlich kleineren Beitrages von den vier Drahtverbindungsbereichen 12a. Der Kelchrand 14 liegt in axialer Projektion zwischen einem Innenkreis E_{I} und einem Außenkreis E_{A}, wobei der Außenkreis E_{A} den Außendurchmesser des Einfangkelches 4 bestimmt und der Innenkreis E_{I} einen Einfangöffnungskreis darstellt, der in guter Näherung die vom Kelchrand 14 berandete Einfangöffnung 7 repräsentiert.

Wenn die vorderen Drahtstücke 12 an ihren Drahtverbindungsbereichen 12a um ca. 180° aufgebogen sind, wie im Beispiel von Fig. 6, ergibt sich ein fast in einer Ebene liegender Kelchrand 4, und die Umfangslänge des Kelchrandes 4 ist dann nur wenig größer als eine Umfangslänge U des Einfangöffnungskreises E_{I}, die sich aus dessen Radius R_{E} durch die Beziehung U=2πR_{E} bestimmt. Mit anderen Worten ist die Umfangslänge U dieses effektiven Einfangöffnungskreises E_{I} nur geringfügig kleiner als die tatsächliche Umfangslänge des Kelchrandes 4, die durch das Produkt 2nL aus der Anzahl 2n der durch die offenen und geschlossenen axialen Schlitze 10, 11 aus dem Rohrstück 2 gebildeten Drahtstücke 12, 13 multipliziert mit der Länge L der offenen axiale Schlitze 10 zuzüglich des Längenbeitrags durch die Drahtverbindungsbereiche 12a gegeben ist.

Somit lässt sich durch Wahl einer genügend großen Länge L der distal offenen Schlitze 10 die Einfangöffnung 7 mit einem gewünscht großen Einfangquerschnitt bereitstellen. Wenn die vorderen Drahtstücke 12 in ihrem Drahtverbindungsbereich 12a um einen kleineren Winkel als 180° aufgebogen werden, ergibt sich mit kleinerem Aufbiegewinkel ein welligerer Verlauf des Kelchrandes 4, wodurch die Kelchrandlänge verglichen mit der Umfangslänge U des effektiven Einfangöffnungskreises E_{I} etwas zunimmt. In vorteilhaften Ausführungsformen wird die Umfangslänge U des Einfangöffnungskreises E_{I} auf einem Wert von mindestens 70% des Produkts 2nL aus der Anzahl 2n der vorderen Drahtstücke 12 und deren Länge L gehalten. Dies führt zu günstigen Kelchformen des Einfangabschnitts 4 mit vergleichsweise großer Einfangöffnung 7 und nur mäßig welligem Verlauf des Kelchrandes 14.

Für den Einfangkelch 4 lassen sich durch Verwendung einer entsprechenden, zum Aufbiegen in den geschlitzten distalen Rohrstückbereich eingebrachten Biegeform beliebige gewünschte Gestaltungen realisieren. Die Fig. 8 bis 10 zeigen drei repräsentative Beispiele. In der
erfindungsgemäßen Ausführungsvariante von Fig. 8 besitzt die Fangdrahtstruktur einen Einfangabschnitt 4a, der sich vom Basisabschnitt 3 aus in distaler Richtung degressiv, d.h. schwächer als linear, aufweitet. Er nimmt dann mit Abstand vor der distalen Ein-fangöffnung 7 einen maximalen Durchmesser D_{E} bzw. Querschnitt ein, von dem aus er sich auf einen etwas kleineren Durchmesser D_{O} bzw. Querschnitt der Einfangöffnung 7 verengt. Das distale Verengen des Einfangkelchs 4a kann ein sicheres Festhalten eines eingefangenen Objektes unterstützen.

Bei einer in Fig. 9 gezeigten Variante, welche nicht Teil der beanspruchten Erfindung ist, erstreckt sich ein Einfangkelch 4b vom Basisabschnitt 3 aus in distaler Richtung mit einer linearen Zunahme seines Aufweitungsdurchmessers. In einer in Fig. 10 dargestellten Ausführungsvariante, welche nicht Teil der beanspruchten Erfindung ist, erstreckt sich ein Einfangkelch 4c ausgehend vom

Basisabschnitt 3 in distaler Richtung mit einer progressiven, d.h. stärker als linearen, Zunahme seines Durchmessers. Es sei erwähnt, dass auch die Kelchvarianten der Fig. 9 und 10 ein sicheres Festhalten eines eingefangenen Objektes dadurch ermöglichen, dass der Einfangkelch 7 so weit in die Umhüllung 6 zurückgezogen wird, bis er sich durch das Einfalten mit seiner Kelchdrahtstruktur eng gespannt gegen das eingefangene Objekt anlegt und dieses mittels Haftreibungskraft am Hinausgleiten nach vorn hindert.

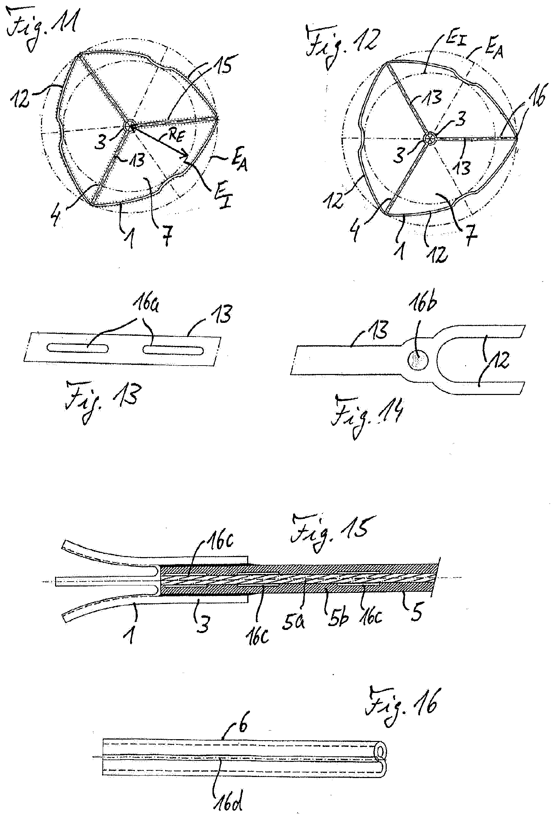

Die Fig. 11 bis 16 veranschaulichen vorteilhafte Ausführungen des erfindungsgemäßen Fangdrahtinstrumentes, die sich besonders für MR-Anwendungen eignen und zu diesem Zweck in an sich bekannter Weise mit einem MR-Markermaterial versehen sind. Fig.11 zeigt hierzu ein Beispiel, bei dem die Fangdrahtstruktur 1 ganzflächig mit einem MR-Markermaterial 15 beschichtet ist, wie durch eine Punktierung der beschichteten Außenflächen der Fangdrahtstruktur 1 angedeutet.

Im Beispiel von Fig. 11 ist der Einfangkelch 4 durch drei hintere Drahtstücke 13 und entsprechend sechs vordere Drahtstücke 12 gebildet, mit entsprechenden Folgen für die Dimensionierung des Kelchrandes 14 in Bezug auf den effektiven Einfangöffnungskreis E_{I} und den Außenkreis E_{A} des Einfangkelchs 4, wie oben zum Ausführungsbeispiel von Fig. 7 für den Fall von n=4 hinteren und acht vorderen Drahtstücken erläutert, worauf verwiesen werden kann.

Fig. 12 zeigt eine Variante des Ausführungsbeispiels von Fig. 11, bei der teilflächige und punktuelle Bereiche der Fangdrahtstruktur 1 mit einem MR-Markermaterial 16 versehen sind. Die Detailansicht von Fig. 13 zeigt eine teilflächige Beschichtung der hinteren Drahtstücke 13 durch eine Reihe von mit Abstand hintereinander angeordneten, länglichen MR-Markerstreifen 16a. Alternativ oder zusätzlich können entsprechende MR-Markerstreifen an den vorderen Drahtstücken 12 vorgesehen sein. Fig. 14 zeigt in einer Detailansicht einen punktuellen MR-Markerfleck 16b, der jeweils im Bereich der Verzweigungsstelle angebracht ist, an der sich das hintere Drahtstück 13 in die zwei vorderen Drahtstücke 12 verzweigt.

Fig. 15 veranschaulicht eine Ausführungsvariante, bei welcher der Zugdraht 5 von einem Drahtkern 5a und einer diesen umgebenden Kernummantelung 5b gebildet ist, wobei Ringe 16c aus einem MR-Markermaterial mit axialem Abstand voneinander auf den Drahtkern 5a aufgebracht und von der Ummantelung 5b eingebettet sind. Fig. 16 zeigt eine Ausführungsvariante, bei welcher die schlauchförmige Umhüllung 6, in welcher der Zugdraht 5 axial beweglich geführt ist, mit einem sich axial erstreckenden, linienförmigen MR-Markermaterial 16d versehen ist. Es versteht sich, dass die zu den Fig. 11 bis 16 erwähnten Maßnahmen zur Bereitstellung einer gewünschten MR-Sichtbarkeit des Fangdrahtinstruments für MR-Anwendungen in beliebiger Weise miteinander kombinierbar sind.

In nicht gezeigten Ausführungsformen der Erfindung weist der Einfangkelch im Bereich seines Kelchkörpers eine stärker verzweigte Struktur auf. Dazu sind dann außer den distal offenen Schlitzen und den mit diesen in axialer Richtung überlappenden, geschlossenen Schlitzen weitere geschlossene Schlitze in das Rohrstück eingebracht, wobei diese weiteren geschlossenen Schlitze axial mit den bereits genannten geschlossenen Schlitzen überlappen und sich in proximaler Richtung über diese hinaus erstrecken. Dabei können sich vorzugsweise wiederum die weiteren geschlossenen axialen Schlitze im Überlappungsbereich in Umfangsrichtung mit den anderen geschlossenen Schlitzen abwechseln. Dies resultiert dann in einer gitterartig verzweigten Struktur des Kelchkörpers.

Wie die obige Beschreibung vorteilhafter Ausführungsbeispiele deutlich macht, stellt die Erfindung ein Fangdrahtinstrument zur Verfügung, das sich insbesondere als medizinisches Fangdrahtinstrument z.B. auch in MR-Anwendungen verwenden lässt und ein sicheres Einfangen von Fremdkörpern, Partikeln etc. von der Frontseite her erlaubt. Dabei wird die Fangdrahtstruktur in fertigungstechnisch vorteilhafter Weise einstückig aus einem Rohrstück hergestellt. Es brauchen dazu keine Drahtverbindungen, wie Schweißstellen, eigens hergestellt werden. Dementsprechend gibt es keine Probleme mit einem Brechen oder einem sonstigen Ausfall entsprechender Schweißstellen oder Klebestellen oder anderweitiger Verbindungsstellen.

## Patentansprüche

1. Fangdrahtinstrument, insbesondere medizinisches Fangdrahtinstrument, mit
- einer durch Schlitzen und Aufbiegen einteilig aus einem Rohrstück (2) gebildeten Fangdrahtstruktur (1), die einen proximalen Basisabschnitt (3) und einen sich davon axial nach vorn erstreckenden distalen Einfangabschnitt (4) aufweist und zwischen einer aus einer Umhüllung (6) nach vorn herausbewegten Einfangsposition mit aufgefaltetem Einfangabschnitt und einer wenigstens teilweise in die Umhüllung zurückbewegten Festhalteposition mit festhaltend eingefaltetem Einfangabschnitt bewegbar ist, und
- einem in der Umhüllung axial beweglich geführten Zugdraht-Betätigungselement (5), wobei die Fangdrahtstruktur an ihrem proximalen Endbereich mit einem distalen Endbereich des Zugdraht-Betätigungselements gekoppelt ist,
**dadurch gekennzeichnet, dass**
- der Einfangabschnitt (4) im aufgefalteten Zustand eine Kelchform mit distaler Einfangöffnung (7) aufweist und dazu ein distaler Endbereich des Rohrstücks (2) mit vom distalen Rohrstückende her eingebrachten, offenen axialen Schlitzen (10) geeigneter Länge und mit zugehörigen zwischenliegenden, mit Abstand vor dem distalen Rohrstückende endenden, geschlossenen axialen Schlitzen (11) größerer Länge als die offenen axialen Schlitze versehen ist, wobei durch die offenen und geschlossenen axialen Schlitze Drahtstücke (12) aus dem Rohrstück gebildet sind, die durch das Aufbiegen des Rohrstücks Umfangsabschnitte der Einfangöffnung bilden,
- weiter **dadurch gekennzeichnet, dass** ein maximaler Durchmesser (D_{E}) der Kelchform des Einfangabschnitts im aufgefalteten Zustand größer ist als ein Durchmesser (Do) der Einfangöffnung.

2. Fangdrahtinstrument nach Anspruch 1, weiter **dadurch gekennzeichnet, dass** je zwei benachbarte Drahtstücke, die aus dem Rohrstück durch die offenen und geschlossenen axialen Schlitze gebildet sind, über einen Krümmungsbereich (12a) zusammenhängen, dessen Radius (R_{D}) mindestens so groß ist wie das Zweieinhalbfache einer Breite (b_{D}) der Drahtstücke im Krümmungsbereich.

3. Fangdrahtinstrument nach Anspruch 1 oder 2, weiter **dadurch gekennzeichnet, dass** eine Umfangslänge eines effektiven Einfangöffnungskreises 70% oder mehr des Produkts (2nL) aus Anzahl (2n) der durch die offenen und geschlossenen axialen Schlitze aus dem Rohrstück gebildeten Drahtstücke (12) multipliziert mit der Länge (L) der offenen axialen Schlitze beträgt.

4. Fangdrahtinstrument nach einem der Ansprüche 1 bis 3, weiter **dadurch gekennzeichnet, dass** die Kelchform des Einfangabschnitts im aufgefalteten Zustand einen oder mehrere Bereiche beinhaltet, in denen der Kelchdurchmesser in distaler Richtung linear, progressiv oder degressiv zunimmt.

5. Fangdrahtinstrument nach einem der Ansprüche 1 bis 4, weiter **dadurch gekennzeichnet, dass** der Einfangabschnitt zweite geschlossene axiale Schlitze im Rohrstück umfasst, die sich nach vorn bis zu einem distalen Ende auf Höhe zwischen einem distalen und einem proximalen Ende der ersten geschlossenen axialen Schlitze und nach hinten bis zu einem proximalen Ende hinter dem proximalen Ende der ersten geschlossenen axialen Schlitze erstrecken.

6. Fangdrahtinstrument nach einem der Ansprüche 1 bis 5, weiter **dadurch gekennzeichnet, dass** die Fangdrahtstruktur und/oder die Umhüllung (6) und/oder das in der Umhüllung axial beweglich geführte Zugdraht-Betätigungselement (5) für die Fangdrahtstruktur ganzflächig oder teilflächig oder punktuell mit einem Magnetresonanz(MR)-Markermaterial (16a, 16b, 16c, 16d) versehen ist.

## Claims

1. Snare instrument, in particular a medical snare instrument, with
- a snare structure (1) which is formed in one piece from a tube section (2), by the latter being slit and bent open, and which has a proximal base portion (3) and, extending axially forward from the latter, a distal snaring portion (4), and which is movable between a snaring position, in which it is moved forward out of an enclosure (6) with the snaring portion folded open, and a securing position, in which it is moved back at least partially into the enclosure with the snaring portion folded in and providing a securing action, and
- a pulling-wire actuation element (5) which is guided axially movably in the enclosure, wherein the proximal end region of the snare structure is coupled to a distal end region of the pulling-wire actuation element,
**characterized in that**
- the snaring portion (4), in the folded-open state, has a cup shape with a distal snare opening (7), and, for this purpose, a distal end region of the tube section (2) is provided with open axial slits (10) of suitable length introduced from the direction of the distal end of the tube section, and, lying between these, with associated closed axial slits (11) which terminate at a distance in front of the distal end of the tube section and are of greater length than the open axial slits, wherein, by means of the open and closed axial slits, wire sections (12) are formed from the tube section and, when the tube section is bent open, the wire sections (12) form circumferential portions of the snare opening,
- further **characterized in that** a maximum diameter (D_{E}) of the cup shape of the snaring portion in the folded-open state is greater than a diameter (Do) of the snare opening.

2. Snare instrument according to Claim 1, further **characterized in that** two adjacent wire sections, formed from the tube section by the open and closed axial slits, are in each case joined together via a region of curvature (12a), of which the radius (R_{D}) is at least as great as two and a half times a width (b_{D}) of the wire sections in the region of curvature.

3. Snare instrument according to Claim 1 or 2, further **characterized in that** a circumferential length of an effective snare opening circle is 70% or more of the product (2nL) of a number (2n) of the wire sections (12) formed from the tube section by the open and closed axial slits, multiplied by the length (L) of the open axial slits.

4. Snare instrument according to one of Claims 1 to 3, further **characterized in that** the cup shape of the snaring portion in the folded-open state comprises one or more regions in which the cup diameter increases linearly, progressively or degressively in the distal direction.

5. Snare instrument according to one of Claims 1 to 4, further **characterized in that** the snaring portion comprises second closed axial slits in the tube section, which slits extend forward as far as a distal end at the level between a distal and a proximal end of the first closed axial slits and rearward as far as a proximal end behind the proximal end of the first closed axial slits.

6. Snare instrument according to one of Claims 1 to 5, further **characterized in that** the snare structure and/or the enclosure (6) and/or the pulling-wire actuation element (5) guided axially movably in the enclosure for the snare structure is provided across the whole surface or a partial surface, or at points, with a magnetic resonance (MR) marker material (16a, 16b, 16c, 16d).

## Revendications

1. Instrument à fil de capture, en particulier instrument à fil de capture à usage médical, ledit instrument comprenant
- une structure à fil de capture (1) qui est formée d'une seule pièce à partir d'une pièce tubulaire (2) par fendage et pliage, qui comporte une portion de base proximale (3) et une portion de capture distale (4) qui s'étend axialement vers l'avant depuis ladite portion de base proximale, et qui peut être déplacée entre une position de capture sortie d'une gaine (6) vers l'avant, la portion de capture étant dépliée, et une position de maintien qui au moins partiellement rétractée dans la gaine, la portion de capture étant repliée pour le maintien, et
- un élément d'actionnement à fil de traction (5) qui est guidé de manière mobile axialement dans la gaine, la structure de fil capture étant accouplée au niveau de sa zone d'extrémité proximale à une zone d'extrémité distale de l'élément d'actionnement à fil de traction,
**caractérisé en ce que**
- la portion de capture (4) a à l'état déplié une forme de coupelle pourvue d'une ouverture de capture distale (7) et pour cela une zone d'extrémité distale de la pièce tubulaire (2) est pourvue de fentes axiales ouvertes (10) de longueur appropriée qui sont ménagées à partir de l'extrémité distale de la pièce tubulaire et de fentes axiales fermées (11) associées, qui sont situées à des positions intermédiaires, qui se terminent à distance en avant de l'extrémité distale de la pièce tubulaire et dont la longueur est supérieure à celle des fentes axiales ouvertes, des pièces de fil (12) étant formés à partir de la pièce tubulaire à travers les fentes axiales ouvertes et fermées et formant des portions périphériques de l'ouverture de capture par dépliage de la pièce tubulaire,
- **caractérisé en outre en ce qu'**un diamètre maximal (D_{E}) de la forme en coupelle de la portion de capture est supérieur à l'état déplié à un diamètre (D_{O}) de l'ouverture de capture.

2. Instrument à fil de capture selon la revendication 1, **caractérisé en outre en ce que** deux pièces de fil adjacentes quelconques, qui sont formées à partir de la pièce tubulaire à travers les fentes axiales ouvertes et fermées, sont contiguës par le biais d'une zone de courbure (12a) dont le rayon (R_{D}) est au moins égal à deux fois et demie une largeur (b_{D}) des pièces de fil dans la zone de courbure.

3. Instrument à fil de capture selon la revendication 1 ou 2, **caractérisé en outre en ce qu'**une longueur circonférentielle d'un cercle d'ouverture de capture efficace est égale à 70 % ou plus du produit (2nL) du nombre (2n) de pièces de fil (12) formées à partir de la pièce tubulaire à travers les fentes axiales ouvertes et fermées multiplié par la longueur (L) des fentes axiales ouvertes.

4. Instrument à fil de capture selon l'une des revendications 1 à 3, **caractérisé en outre en ce que** la forme en coupelle de la portion de capture à l'état déplié comprend une ou plusieurs zones dans lesquelles le diamètre de la coupelle augmente linéairement, progressivement ou dégressivement dans la direction distale.

5. Instrument à fil de capture selon l'une des revendications 1 à 4, **caractérisé en outre en ce que** la portion de capture comprend, dans la pièce tubulaire, des deuxièmes fentes axiales fermées qui s'étendent vers l'avant jusqu'à une extrémité distale à une hauteur située entre une extrémité distale et une extrémité proximale des premières fentes axiales fermées et qui s'étendent vers l'arrière jusqu'à une extrémité proximale en arrière de l'extrémité proximale des premières fentes axiales fermées.

6. Instrument à fil de capture selon l'une des revendications 1 à 5, **caractérisé en outre en ce que** la structure de fil de capture et/ou la gaine (6) et/ou l'élément d'actionnement à fil de traction (5) guidé de manière mobile axialement dans la gaine et destiné à la structure de fil de capture est pourvue, sur toute la surface ou une partie de la surface ou ponctuellement, d'un matériau marqueur à résonance magnétique (MR) (16a, 16b, 16c, 16d).
